(19) Europäisches Patentamt
European Patent Office
Office européen des brevets

(11) Numéro de publication: 0 044 091 B1

# (12) FASCICULE DE BREVET EUROPEEN

(45) Date de publication du fascicule du brevet: **11.01.84**

(51) Int. Cl.³: **C 07 C 97/10** // C07C103/50

(21) Numéro de dépôt: **81107894.8**

(22) Date de dépôt: **20.09.79**

(60) Numéro de publication de la demande initiale en application de l'article 76 CBE:

(54) **Procédé de préparation de N-alcoylamino benzophénones.**

(30) Priorité: **25.09.78 FR 7827401**

(43) Date de publication de la demande: **20.01.82 Bulletin 82/3**

(45) Mention de la délivrance du brevet: **11.01.84 Bulletin 84/2**

(84) Etats contractants désignés: **AT BE CH DE GB IT LU NL SE**

(56) Documents cités:
**FR - A - 2 081 393**
**FR - A - 2 436 774**

**COMPTES RENDUS HEBDOMADAIRES DES SEANCES DE L'ACADEMIE DES SCIENCES vol. 285, no. 5, Série C, 19 septembre 1977, J. RENAULT et al.: "Application de la catalyse par transfert de phase à l'alkylation des hydroxy-4 quinoléines", pages 199-201**

**Le dossier contient des informations techniques présentées postérieurement au dépôt de la demande et ne figurant pas dans le présent fascicule.**

(73) Titulaire: **PIERRE FABRE S.A., 125, rue de la Faisanderie, F-75116 Paris (FR)**

(72) Inventeur: **Mouzin, Gilbert, 21, rue Sainte Foy, F-81100 Castres (FR)**
Inventeur: **Cousse, Henri, La Foun de Los Nobios Chemin de Lastinos, F-81100 Castres (FR)**
Inventeur: **Stenger, Antoine, 11, rue des Cigales, F-81100 Castres (FR)**

(74) Mandataire: **Corre, Jacques Denis Paul et al, Cabinet Regimbeau 26, Avenue Kléber, F-75116 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 044 091 B1

BUNDESDRUCKEREI BERLIN

# Procédé de préparation de N-alcoylamino benzophénones

La présente invention concerne un nouveau procédé de préparation de dérivés de N-alcoylamino benzophénones répondant à la formule générale I

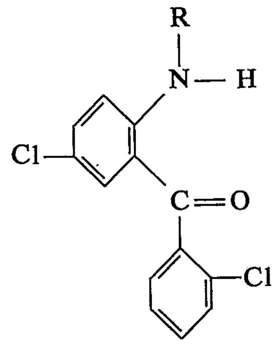

(I)

dans laquelle:

R représente un groupe alcoyle inférieur.

Dans la publication Comptes Rendus Hebdomadaires des Séances de l'Académie des Sciences, vol. 285, n° 5, Série C, 19 septembre 1977, il est fait état d'un procédé d'alcoylation de 4-hydroxy quinoléines faisant intervenir une catalyse par transfert de phase. Ce procédé antérieur présente cependant l'inconvénient fondamental de ne pas être sélectif et sortout dans le cas de l'amino-alkylation de ne pas respecter le caractère aromatique du fragment pyridique.

FR-A-n° 2 081 393 décrit un procédé permettant notamment de préparer des monoalcoylaminebenzophénones. Ce procédé ne peut cependant pas être réalisé en une seule étape, étant donné que l'opération d'alcoylation doit être nécessairement précédée d'une étape d'acylation et le cas échéant suivie d'une étape de saponification.

La présente invention a précisément pour but de remédier aux inconvénients de la technique antérieure en fournissant un procédé de synthèse comportant une seule étape opérationnelle, ayant une parfaite sélectivité d'alcoylation et conduisant à un rendement élevé de monoalcoylaminebenzophénones.

Le procédé objet de la présente invention est du type selon lequel on fait réagir l'amino-2 dichloro-2'-5 benzophénone de formule II

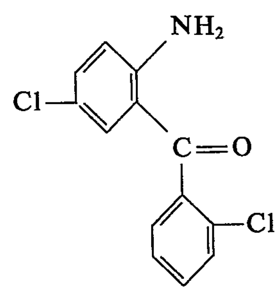

(II)

avec un sulfate d'alcoyle inférieur de formule $SO_4(R)_2$ où R représente un radical alcoyle inférieur, en présence de soude.

Le procédé objet de la présente invention est caractérisé en ce que la réaction est mise en oeuvre dans un solvant organique en présence d'un catalyseur constitué par le bromure de tétrabutylammonium.

Le procédé objet de la présente demande permet en particulier de préparer des intermédiaires de synthèse nécessaires à la préparation de dérivés d'ortho chloro benzoyl-2 chloro-4 glycylanilide protégés par la demande de brevet européen n° 79 400 668.4.

A titre d'exemple on mentionnera ci-après la préparation du N-N' diméthyl N(hydroxy-2 éthyl) ortho chloro benzoyl-2' chloro-4' glycylanilide.

a) Préparation du N-méthyl benzoyl-2' dichloro-2''-4' bromo-2 acétanilide

A une solution de 56 g de méthyl amino-2 dichloro-2'-5 benzophénone dans 400 $cm^3$ d'acétate d'éthyle, on ajoute un volume égal de glace, puis 23 $cm^3$ de bromure de bromacétyle. Après une nuit

d'agitation à température ambiante, on ajoute 300 $cm^3$ d'éther éthylique, on décante, on lave à la soude 2 N et on lave à l'eau jusqu'à neutralité.

On sèche ensuite sur sulfate de sodium, on filtre et l'on évapore jusqu'à siccité. Le résidu est traité à l'éther de pétrole puis recristallisé dans l'acétate d'éthyle. On récupère ainsi 65 g de cristaux, rendement 81%.

Point de fusion: 86° C
Chromatographie sur plaque:
support: gel de silice 60 F 254 Merck
solvant: acétate d'éthyle-éther de pétrole 30/70
révélation: UV et iode
Rf: 0,33.

b) Préparation de N-N' diméthyl N(hydroxy-2 éthyl) ortho chloro benzoyl-2' chloro-4' glycylanilide

A une solution de 60,15 g (0,15 mole) de N-méthyl ortho chloro benzoyl-2' chloro-4' bromo-2 acétanilide dans 500 $cm^3$ d'acétone, on ajoute 25 $cm^3$ (0,31 mole) de méthyl amino-2 éthanol et l'on chauffe 2 heures à reflux.

On évapore la solution jusqu'à siccité, on reprend le résidu à l'acide chlorhydrique N et on lave à l'éther. La phase aqueuse acide est traitée au bicarbonate de sodium, puis on extrait à l'éther et on lave à l'eau jusqu'à neutralité.

On sèche sur sulfate de sodium, on filtre et l'on évapore la phase organique. Le résidu obtenu est recristallisé dans l'hexane-acétate d'éthyle. On récupère ainsi, avec un rendement de 80%, le produit de formule:

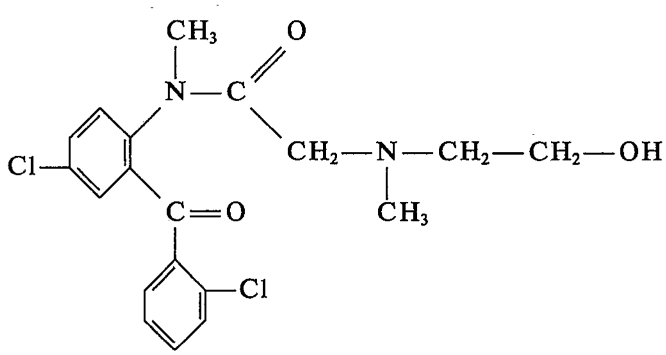

Formule brute: $C_{19}H_{20}Cl_2N_2O_3$
Masse moléculaire: 395,27
Cristaux: blanc cassé
Point de fusion: 79° C
Chromatographie sur plaque:
support: gel de silice 60 F 254 Merck
solvant: butanol-acide acétique-eau 6/2/2
révélation: UV et iode
Rf: 0,41.

Le méthyl amino-2 dichloro-2'-5 benzophénone de départ est précisément obtenu par la mise en oeuvre du procédé selon la présente invention par réaction d'amino-2 dichloro-2'-5 benzophénone avec du sulfate de méthyle dans un solvant organique contenant de la soude et du bromure de tétrabutylammonium en tant que catalyseur.

**Revendication**

Procédé de préparation de N-alcoylamino benzophénones, répondant à la formule générale I

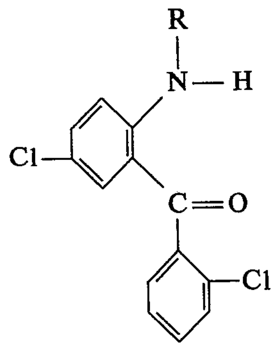

(I)

dans laquelle
R représente un groupe alcoyle inférieur,
du type selon lequel on fait réagir l'amino-2 dichloro-2'-5 benzophénone de formule II

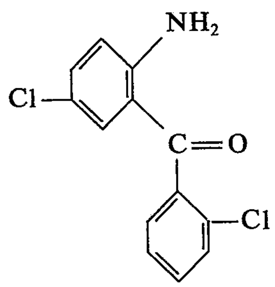

(II)

avec un sulfate d'alcoyle inférieur de formule $SO_4(R)_2$ où R représente un radical alcoyle inférieur, en présence de soude, caractérisé en ce que la réaction est mise en oeuvre dans un solvant organique en présence d'un catalyseur constitué par le bromure de tétrabutylammonium.

**Patentanspruch**

Verfahren zur Herstellung von N-Alkylaminobenzophenonen der allgemeinen Formel (I)

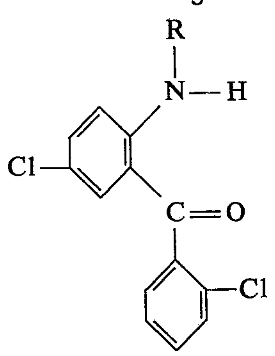

(I)

worin
R eine nied. Alkylgruppe bedeutet,
wobei man 2-Amino-2'-dichlor-5-benzophenon der Formel (II)

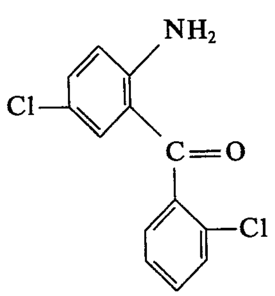

(II)

mit einem nied. Alkylsulfat der Formel $SO_4(R)_2$, worin R eine nied. Alkylgruppe darstellt, in Anwesenheit von Soda reagieren läßt, dadurch gekennzeichnet, daß man die Reaktion in einem organischen Lösungsmittel in Anwesenheit eines aus Tetrabutylammoniumbromid bestehenden Katalysators durchführt.

**Claim**

Process for the preparation of N-alkoylamino benzophénones, having the general formula I

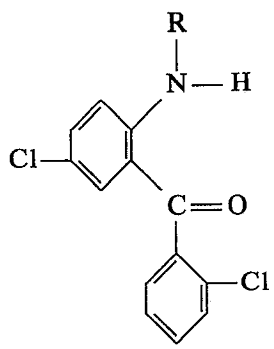

(I)

in which
R represents a lower alkoyl group,
of the type consisting of the reaction of the amino-2 dichloro-2'-5 benzophénone of the formula II

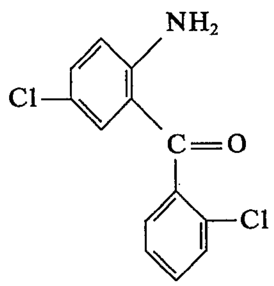

(II)

with a lower alkoyl sulphate of the formula $SO_4(R)_2$ where R represents a lower alkoyl radical, in the presence of caustic soda, characterized in that the reaction is carried out in an organic solvent in the presence of a catalyst constituted by the tetrabutylammonium bromid.